# EUROPEAN PATENT APPLICATION

(11) **EP 2 730 559 A1**
(43) Date of publication of application: **14.05.2014**
(21) Application number: 12807516.5
(22) Date of filing: 04.07.2012
(51) Int. Cl.: C07C 233/65, A61K 31/192

(54) **DEUTERATED PHENYLPROPIONIC ACID DERIVATIVE**

(30) Priority: 05.07.2011 US 201161504374 P
(71) Applicant: Research Foundation Itsuu Laboratory, Tokyo 158-0094 (JP)
(72) Inventor: MURATAKE, Hideaki, Machida-shi Tokyo 194-0044 (JP); SHUDO, Koichi, Tokyo 168-0073 (JP); AMANO, Yohei, Kawasaki-shi Kanagawa 214-0014 (JP); TODA, Takahiro, Tokyo 158-0094 (JP)
(74) Representative: Godemeyer Blum Lenze - werkpatent
(86) International application number: PCT/JP2012/067033
(87) International publication number: WO 2013/005753

(57) **Abstract**

A compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof, and a prodrug for releasing Am80 as an active medicament after being absorbed into a living body, which comprises the aforementioned compound, a salt thereof, or an ester thereof as an active ingredient.

## Description

### Technical Field

The present invention relates to a deuterated phenylpropionic acid derivative that can release a retinoid as an active medicament after it is absorbed into a living body.

### Background Art

Activity and effect of a medicament are usually determined according to the blood concentration (Cmax and Tmax) and half-life thereof (t1/2), the area under the blood concentration-time curve (AUC), or the like. These pharmacokinetic parameters are controlled by various methods, and there are several researches concerning means for controlling a pharmacokinetic parameter by substituting a deuterium atom for a hydrogen atom contained in a medicament at a specific position.

Medicaments in which a hydrogen atom is substituted with a deuterium atom at a specific position sometimes give a prolonged half-life (t1/2) of the active compound in the medicament as compared to the medicament not containing deuterium atom (protium compound), and deuterated compounds may sometimes achieve superior i*n vivo* kinetics as a medicament compared with corresponding protium compounds.

The same possibility may also be expected for a prodrug of a medicament, and it can be expected that blood concentration of an active medicament can be controlled by extending half-life of a prodrug thereof. Specifically, release of an active medicament from a deuterated prodrug after administration may be delayed compared with that from the prodrug as a protium compound, and thus rapid increase of the blood concentration of the active medicament may be suppressed, and the maximum blood concentration may also be lowered. Further, when metabolic pathway of the medicament consists of two or more stages, generation of intermediate metabolites may also be suppressed. It is considered that achieving such delay of release of an active medicament and suppression of generation of intermediate metabolites is preferred for maintaining the effectiveness, as well as avoiding at least a part of side reactions. However, any deuterated medicament has not been put into practical use so far, and any development of a deuterated prodrug as a pharmaceutical product has not been known.

Retinoic acid (vitamin A acid) is an active metabolite of vitamin A, and has extremely important physiological functions, e.g., inducing differentiation of immature cells under development processes toward mature cells having specific functions, acceleration of cell proliferation, and life support action. It has been revealed that various vitamin A derivatives synthesized so far also have similar physiological functions, and such derivatives include, for example, the benzoic acid derivatives disclosed in Japanese Patent Unexamined Publication (KOKAI) Nos. (Sho)61-22047/1986 and (Sho)61-76440/1986, and the compounds described in Journal of Medicinal Chemistry, 1988, Vol. 31, No. 11, p.2182. Retinoic acid and the aforementioned compounds having retinoic acid-like biological activities are generically called "retinoids".

For example, it has been proved that all-trans retinoic acid binds as a ligand to the retinoic acid receptor (RAR) present in cellular nucleus, which belongs to the intranuclear receptor super family (Evans, R.M., Science, 240, p.889, 1988), and regulates proliferation and differentiation of animal cells or cellular mortalities (Petkovich, M., et al., Nature, 330, pp.444-450, 1987). In addition, as for the expression of physiological activities of retinoic acid, the existence of retinoid X receptor (RXR of which ligand is 9-cis-retinoic acid) has been elucidated. The retinoid X receptor has been revealed to control the expression of the physiological activities of the retinoic acid by inducing or suppressing the transcription of a target gene by forming a dimer with the retinoic acid receptor (RAR) (Mangelsdorf, D. J. et al., Nature, 345, pp.224-229).

It has also been suggested that the aforementioned compounds having the retinoic acid-like biological activities (e.g., 4-[(5,6,7,8-tetrahydro-5,5,8,8-tetramethyl-2-naphthalenyl)carbamoyl]benzoic acid (Am80) and the like) also bind to RAR in similar manners to retinoic acid to exhibit their physiological actions (see, Hashimoto, Y., Cell Struct. Funct., 16, pp.113-123, 1991; Hashimoto, Y., et al., Biochem. Biophys. Res. Commun., 166, pp.1300-1307, 1990). Clinically, these compounds were found to be useful for therapeutic and preventive treatments of vitamin A deficiency disease, hyperkeratosis of epithelial tissue, rheumatism, delayed allergy, bone diseases, leukemia and certain types of cancer, and Am80 is clinically used as a therapeutic agent for acute promyelocytic leukemia (tamibarotene, "Amnolake Tablets", Toko Pharmaceutical Industrial Co., Ltd.).

### Prior art references

### Non-patent documents

Non-patent document 1: Cell Struct. Funct., 16, pip.113-123, 1991
Non-patent document 2: Biochem. Biophys. Res. Commun., 166, pp.1300-1307, 1990

### Disclosure of the Invention

### Object to be achieved by the invention

An object of the present invention is to provide a deuterated prodrug that is a compound capable of releasing a retinoid as an active medicament after it is absorbed into a living body.

### Means for achieving the object

The inventors of the present invention found that when a part or all of hydrogen atoms in the propionic acid moiety of 3-[4-[(5,6,7,8-tetrahydro-5,5,8,8-tetramethyl-2-naphthalenyl)carbamoyl]phenyl]propionic acid, that functions as a prodrug capable of releasing Am80 in a living body (this non-deuterated compound may be henceforth referred to as "protium compound" or "protium compound propionic acid derivative"), were replaced with deuterium atoms, release of Am80 from the resulting deuterated compound (this deuterated compound may be henceforth referred to as "deuterated propionic acid derivative", examples thereof include and the like) after administration was delayed compared with that from the protium compound, and the deuterated propionic acid derivative had superior characteristic features as a prodrug concerning the pharmacokinetic parameters such as maximum blood concentration, blood concentration half-life (t1/2) and area under the blood concentration-time curve (AUC) compared with the protium compound. The present invention was accomplished on the basis of the aforementioned finding.

The present invention thus provides a compound corresponding to 4-[(5,6,7,8-tetrahydro-5,5,8,8-tetramethyl-2-naphthalenyl)carbamoyl]phenyl-3-propionic acid in which a part or all of hydrogen atoms in the ethylene group constituting the propionic acid moiety are replaced with deuterium atoms, a salt thereof, or an ester thereof.

According to preferred embodiments of the present invention, there are provided 3-[4-[(5,6,7,8-tetrahydro-5,5,8,8-tetramethyl-2-naphthalenyl)carbamoyl]-phenyl]propionic acid-2,2,3,3-d4, 3-[4-[(5,6,7,8-tetrahydro-5,5,8,8-tetramethyl-2-naphthalenyl)carbamoyl]phenyl]propionic acid-2,2-d2, 3-[4-[(5,6,7,8-tetrahydro-5,5,8,8-tetramethyl-2-naphthalenyl)carbamoyl]phenyl]propionic acid-3,3-d2, a salt thereof, or an ester thereof.

As another aspect of the present invention, there is provided a prodrug containing a compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof as an active ingredient. This prodrug can release Am80 as an active medicament, after it is absorbed into a living body.

Therefore, the present invention provides use of a compound corresponding to 3-[4-[(5,6,7,8-tetrahydro-5,5,8,8-tetramethyl-2-naphthalenyl)carbamoyl]phenyl]-propionic acid in which a part or all of hydrogen atoms in the ethylene group constituting the propionic acid moiety are replaced with deuterium atoms, a salt thereof, or an ester thereof as a prodrug. The present invention also provides use of the aforementioned prodrug for releasing Am80 as an active medicament.

As a further aspect of the present invention, there is provided a method comprising the step of administering a compound corresponding to 3-[4-[(5,6,7,8-tetrahydro.5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof to a mammal including human to induce generation of Am80 in the living body of the mammal.

The present invention also provides a method comprising administering a compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof to a mammal including human to induce generation of Am80 in the living body of the mammal, and performing prophylactic treatment and/or therapeutic treatment of a disease preventable and/or treatable with Am80 by using Am80 generated in the living body as an active ingredient.

### Effect of the Invention

The deuterated propionic acid derivative provided by the present invention shows delayed release of Am80 after administration thereof compared with the protium compound propionic acid derivative, and has superior characteristic features as a prodrug concerning pharmacokinetic parameters such as the maximum blood concentration, the blood concentration half-life (t1/2) and the area under the blood concentration-time curve (AUC) compared with the protium compound. Therefore, the deuterated propionic acid derivative provided by the present invention can be used as, for example, a prodrug as a sustained release drug of Am80 as an active medicament, and it is useful as a prodrug that can continuously exhibit the action of Am80 over a long period of time. The sustained release drug also makes it possible to suppress the maximum blood concentration and thereby reduce side reactions. Furthermore, since deuterium itself is substantially harmless to a living body, the deuterated propionic acid derivative is safe at the same level as that of the protium compound, and it does not metabolically generate any metabolic products harmful to a living body.

### Modes for Carrying out the Invention

The present invention provides a compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof.

Although type of the compound in which a part or all of hydrogen atoms in the ethylene group constituting the propionic acid moiety are replaced with deuterium atoms is not particularly limited, examples include, for example, compounds represented as A-CHD-CH₂-COOH, A-CH₂-CHD-COOH, A-CD₂-CH₂-COOH, A-CHD-CHD-COOH, A-CH₂-CD₂-COOH, A-CD₂-CHD-COOH, A-CHD-CD₂-COOH, A-CD₂-CD₂-COOH, or the like, wherein A is the residue of the compound except for the propionic acid moiety (A is 3-[4-[(5,6,7,8-tetrahydro-5,5,8,8-tetramethyl-2-naphthalenyl)-carbamoyl]phenyl] group). Although the deuterated propionic acid derivative provided by the present invention may consist of a single kind of the deuterated propionic acid derivative, or a mixture of two or more kinds of the deuterated propionic acid derivatives. Particularly preferred examples include A-CD₂-CH₂-COOH, A-CD₂-CD₂-COOH, and a mixture thereof.

The deuterated propionic acid derivative provided by the present invention may form a base addition salt, and may exists as a metal salt such as sodium salt, potassium salt, magnesium salt, or calcium salt, an ammonium salt, an organic amine salt such as triethylamine salt or ethanolamine salt, or the like. Among such salts as mentioned above, a physiologically acceptable salt can be used as the prodrug. Moreover, arbitrary hydrates and solvates of the compound in the form of a free acid or a salt are also encompassed within the scope of the present invention.

Furthermore, the carboxyl group of the propionic acid moiety of the deuterated propionic acid derivative may form an ester. As the ester, a physiologically acceptable ester is preferred. Preferred examples of the ester residue include, for example, methyl group, ethyl group, propyl group, isopropyl group, butyl group, isobutyl group, t-butyl group, benzyl group, acetoxymethyl group, 1-(acetoxy)ethyl group, propionyloxymethyl group, 1-(propionyloxy)ethyl group, butyryloxymethyl group, 1-(butyryloxy)ethyl group, isobutylyloxymethyl group, 1-(isobutyryloxy)ethyl group, valeryloxymethyl group, 1-(valeryloxy)ethyl group, isovaleryloxymethyl group, 1-(isovaleryloxy)ethyl group, pivaloyloxymethyl group, 1-(pivaloyloxy)ethyl group, methoxycarbonyloxymethyl group, 1-(methoxycarbonyloxy)ethyl group, ethoxycarbonyloxymethyl group, 1-(ethoxycarbonyloxy)ethyl group, propoxycarbonyloxymethyl group, 1-(propoxycarbonyloxy)ethyl group, isopropoxycarbonyloxymethyl group, 1-(isopropoxycarbonyloxy)ethyl group, butoxycarbonyloxymethyl group, 1-(buthoxycarbonyloxy)ethyl group, isobutoxycarbonyloxymethyl group, 1-(isobuthoxycarbonyloxy)ethyl group, t-buthoxycarbonyloxymethyl group, 1-(t-buthoxycarbonyloxy)ethyl group, cyclopentanecarbonyloxymethyl group, 1-(cyclopentanecarbonyloxy)ethyl group, cyclohexanecarbonyloxymethyl group, 1-(cyclohexanecarbonyloxy)ethyl group, cyclopenthyloxycarbonyloxymethyl group, 1-(cyclopenthyloxycarbonyloxy)ethyl group, cyclohexyloxycarbonyloxymethyl group, 1-(cyclohexyloxycarbonyloxy)ethyl group, benzoyloxymethyl group, 1-(benzoyloxy)ethyl group, phenoxycarbonyloxymethyl group, 1-(phenoxycarbonyloxy)ethyl group, (5-methyl-2-oxo-1,3-dioxolen-4-yl)methyl group, 2-trimethylsilylethyl group, and the like, but are not limited to these examples.

The preparation method of the compound of the present invention is not particularly limited, and hydrogen atoms of the ethylene group of the propionic acid moiety in an arbitrary corresponding protium compound propionic acid derivative can be replaced with deuterium atoms by, for example, allowing a small amount of hydrogen molecules (H₂) to act on the protium compound propionic acid derivative in the presence of a metal catalyst and a deuterated solvent according to the method of Sajiki et al. (Sajiki et al., Tetrahedron Letters, 46, pp.6995-6998, 2005; Journal of Synthetic Organic Chemistry, Japan, 65, pp.1179-1189, 2007). The reaction can be performed, for example, at a temperature of about 80 to 150°C for several hours to several days. Structure and substitution position of deuterium in the deuterated compound can be easily confirmed by ¹H-NMR and ¹³C-NMR. Although deuteration rate is not particularly limited, it is, for example, 90% or higher, preferably 95% or higher, more preferably 98% or higher, and the compound of a further higher deuteration rate can be prepared by appropriately repeating the aforementioned reaction as required.

As the catalyst, a metal catalyst used for usual catalytic hydrogenation such as palladium catalyst, platinum catalyst, nickel catalyst, cobalt catalyst or iridium catalyst can be used, and a catalyst comprising such a metal catalyst as mentioned above carried on an inert carrier such as activated carbon and an inactive inorganic compound can be preferably used. A palladium/carbon catalyst and the like can be preferably used.

Examples of the deuterated solvent include, for example, deuterated water (D₂O), organic solvents including deuterated alcohols such as deuterated methanol, deuterated ethanol, deuterated propanol, deuterated isopropanol, deuterated butanol, deuterated tert-butanol, deuterated pentanol, deuterated hexanol, and deuterated heptanol, deuterated carboxylic acids such as deuterated formic acid, deuterated acetic acid, deuterated propionic acid, deuterated butyric acid, deuterated isobutyric acid, deuterated valeric acid, deuterated isovaleric acid, and deuterated pivalic acid, and the like. Although deuteration rate of these deuterated solvents is not particularly limited, a deuterated solvent having a deuteration rate of, for example, 90% or higher, preferably 95% or higher, more preferably 98% or higher, is preferably used.

Among these solvents, deuterated water is preferably used from the viewpoints of environmental aspect, workability, and the like. Further, in addition to a deuterated solvent, an aprotic solvent or a hydrophobic solvent can also be added. It is also possible to use a partially deuterated solvent such as CH₃OD and C₂H₅OD depending on the reaction conditions. Since deuteration rate of marketed deuterated water is about 99 to 99.9%, the deuterated propionic acid derivative can be generally produced at a deuteration rate of about 95 to 99% by using a metal catalyst, deuterated water, and a catalytic amount of hydrogen molecules, and if deuterium molecules (D₂) is used instead of hydrogen molecules, the target compound can be produced with a still higher deuteration rate. In general, when the deuterated propionic acid derivative of the present invention is used as a prodrug, desired characteristics can fully be demonstrated with a deuteration rate of 90% or higher.

For example, by condensing aminonaphthalene and a protected or non-protected deuterated 3-(4-carboxyphenyl)propionic acid in a conventional manner, the deuterated propionic acid derivative of the present invention can be prepared. For example, deuterated 3-(4-carboxyphenyl)propionic acid-2,2,3,3-d4 can be prepared by stirring a corresponding protium compound of 3-(4-carboxyphenyl)propionic acid, 4-carboxycinnamic acid, or 4-carboxyphenylacetylenecarboxylic acid in deuterated water with heating in the presence of a palladium or platinum catalyst and a catalytic amount of hydrogen molecules.

In this reaction, if the deuteration conditions are appropriately chosen, hydrogen atoms on the aromatic ring are hardly replaced with deuterium atoms. For example, by heating sodium salt of 3-[4-[(5,6,7,8-tetrahydro-5,5,8,8-tetramethyl-2-naphthalenyl)carbamoyl]phenyl]propionic acid with a palladium catalyst in deuterated water in the presence of hydrogen molecules, there can be obtained 3-[4-[(5,6,7,8-tetrahydro-5,5,8,8-tetramethyl-2-naphthalenyl)carbamoyl]phenyl]propionic acid as well as 3-[4-[(5,6,7,8-tetrahydro-5,5,8,8-tetramethyl-2-(3-d)naphthalenyl)carbamoyl]-phenyl]propionic acid-2,2,3,3-d4. A compound deuterated in a moiety other than the ethylene group constituting the propionic acid moiety such as the above latter compound is also encompassed within the scope of the present invention. For example, the 3-deuterated derivative can be produced by condensing 4-carboxybenzyl bromide-d2 obtained from 4-CD₃-benzoic acid and an acetic acid ester, and the 2-deuterated derivative can be produced by decarboxylating a Meldrum's acid derivative in deuterated water.

If the deuterated propionic acid derivative of the present invention is orally or parenterally administered as a prodrug to a mammal including human, the propionic acid or an ester thereof is metabolized and thereby converted into carboxyl group in the living body of the mammal, and Am80 as an active medicament is released in the living body. Therefore, the deuterated propionic acid derivative of the present invention is useful as a prodrug for releasing Am80 in a living body.

It has been found that Am80 is useful for therapeutic and prophylactic treatments of vitamin A deficiency disease, hyperkeratosis of epithelial tissue, rheumatism, delayed allergy, bone diseases, leukemia, liver cancer, and the like, and is already clinically used as a therapeutic agent for acute promyelocytic leukemia. Further, for Am80, there have been reported, for example, an action of improving memory consolidation in neurodegenerative diseases such as Alzheimer disease, Parkinson's disease, schizophrenia, drug dependence, and abnormality of autonomic nerve, a prophylactic and/or therapeutic action for inflammatory bowel diseases including graft versus host disease, ulcerative colitis, and Crohn's disease, a prophylactic and/or therapeutic action for impaired secretion diseases accompanied by lymphocytic infiltration into secretory glands including type I diabetes mellitus and Sjoegren's syndrome, a prophylactic and/or therapeutic action for eye diseases resulting from increased blood vessel permeability such as diabetic retinopathy, and age-related macular degeneration, an action of improving physical dysfunctions such as motor dysfunction resulting from nerve injury induced by an accident, cerebral apoplexy, or the like, and a prophylactic and/or therapeutic action for lower urinary tract diseases resulting from lower urinary tract obstructions including interstitial cystitis, cystalgia syndrome, overactive bladder, and the like, and Am80 has prophylactic and/or therapeutic actions against these diseases. Therefore, the prodrug of the present invention can be used for attaining the prophylactic and/or therapeutic actions of Am80 as an active medicament in living bodies against the aforementioned diseases preventable and/or treatable with Am80.

As the prodrug, one or more kinds of substances selected from the group consisting of the deuterated propionic acid derivative, a salt thereof, a hydrate and a solvate of these can be used. Although the aforementioned substances per se may be administered as the prodrug, they can be preferably administered as a pharmaceutical composition for oral or parenteral administration producible by the methods well known to those skilled in the art. Examples of pharmaceutical composition suitable for oral administration include, for example, tablets, capsules, powders, subtilized granules, granules, solutions, syrups, and the like, and examples of pharmaceutical composition suitable for parenteral administration include, for example, injections, suppositories, inhalants, eye drops, nose drops, and the like.

The aforementioned pharmaceutical composition can be prepared with pharmacologically and pharmaceutically acceptable additives. Example of the pharmacologically and pharmaceutically acceptable additives include, for example, excipients, disintegrating agents and disintegrating aids, binders, lubricants, coating agents, dyes, diluents, bases, dissolving agents and dissolving aids, isotonic agents, pH modifiers, stabilizers, propellants, tackifiers, and the like.

Dose of the prodrug is not particularly limited, and can be appropriately chosen with reference to doses of Am80 as the active medicament used for various diseases, and it can be appropriately increased or decreased according to various factors that should usually be taken into consideration, such as body weight and age of patients, type and symptoms of diseases, and route of administration. The dose can generally be appropriately chosen with reference to the dose of Am80 with taking absorption efficiency and metabolic efficiency of the prodrug into consideration. For oral administration, for example, it can be used at a daily dose for adults in the range of about 0.01 to 1,000 mg.

### Examples

Hereafter, the present invention will be still more specifically explained with reference to examples. However, the scope of the present invention is not limited by the following examples.

### Example 1: Deuteration of methyl 3-(4-carboxyphenyl)propionate

Methyl 3-(4-carboxyphenyl)propionate (365 mg) was suspended in deuterated water (D₂O; deuteration rate, 99.9%; 10 ml) containing 1/2 equivalent of sodium carbonate, the suspension was added with 10% Pd/C (37 mg), and after the space in the reaction vessel (about 10 ml) was filled with hydrogen gas (H₂), and sealed with a stopper, the mixture was sufficiently stirred at 110 to 140°C. The reaction mixture was filtered through Celite, and extracted with chloroform and methanol (yield, 360 mg).

### Example 2: Deuteration of 3-(4-carboxyphenyl)propionic acid

Deuterated water (40 ml) containing 3-(4-carboxyphenyl)propionic acid (2.0 g), less than 1/2 equivalent of sodium carbonate, and 10% Pd/C (200 mg) was stirred with heating at 125°C for 48 hours in the presence of hydrogen gas (100 ml) in a sealed container. The reaction mixture was once filtered through Celite, then Pd/C was supplemented, the space in the container was replaced with hydrogen gas, and the reaction was performed under the same conditions. The reaction was performed 3 times in total. The reaction mixture was neutralized to obtain the objective deuterated dicarboxylic acid (1.93 g). Although this carboxylic acid was hardly soluble, it could be confirmed that the ratio of protons on the carbons at the 2- and 3-positions was less than 1% on the basis of the results of NMR (1% Na₂CO₃/D₂O). It could be confirmed that the hydrogen atoms on the benzene ring were not replaced on the basis of the results of NMR performed for the product of the reaction with 4-aminonaphthalene.
HRMS: Calcd for C₁₀H₆D₄O₄ 198.0830, Found 198.0825
MS (m/z): 198 (M+, 67), 181 (11), 152 (50), 137 (61), 109 (100), 83 (19), 79 (25), 45 (28) NMR (1% Na₂CO₃·D₂D): 2.35 (0.02H, br.s), 2.79 (0.01H, br.s), 7.22 (2H, J=8), 7.68 (2H, J=8).

If deuterated hydrogen gas (D₂ gas) is used in the aforementioned reaction instead of hydrogen gas, the objective substance can be prepared with a still higher deuteration rate. If the catalyst is deactivated, or the hydrogen gas or deuterated hydrogen gas disappears due to oxidization or the like, the deuteration rate may decrease. In such a case, the objective substance can be obtained at a desired deuteration rate by repeating the aforementioned reaction in deuterated water.

The same dicarboxylic acid can be obtained by performing the reaction with 4-carboxycinnamic acid or 4-carboxyphenylpropargyl acid in deuterated water in the presence of a catalytic amount of hydrogen gas or deuterated hydrogen gas so that reduction simultaneously occurs.

### Example 3: Methylation of 3-(4-carboxyphenyl)(2-d,d-3-d,d)propionic acid

The dicarboxylic acid (1.9 g) obtained in Example 2 mentioned above was suspended in methanol (30 ml), the suspension was added with a catalytic amount of thionyl chloride (14 µl, 2 mol %), and the mixture was stirred at room temperature for 16 hours. Methanol was evaporated, the residue was extracted with 10% aqueous sodium carbonate, and the extract was made acidic with concentrated hydrochloric acid. The deposited crystals were collected by filtration to obtain methyl 3-(4-carboxyphenyl)(2-d,d-3-d,d)propionate (1.8 g). A small amount of the starting material dicarboxylic acid was collected, and by-production of a diester compound was also confirmed.
HRMS: Calcd for C₁₁H₈D₄O₄ 212.0986, Found 212.1001
MS(m/z): 212 (M+,34), 195 (4), 181 (12), 152 (100), 137 (40), 134 (18), 123 (15), 109 (76), 79 (16)
NMR (CDCl₃): 2.65 (0.02H, br.s), 3.01 (0.02H, br.s), 3.67 (3H, s), 7.32 (2H, J=8.5), 8.04 (2H, J=8.5)

### Example 4: Methyl 3-[4-[(5,6,7,8-tetrahydro-5,5,8,8-tetramethyl-2-naphthalenyl)carbamoyl]phenyl]propionate-2,2,3,3-d4

Methyl 3-(4-carboxyphenyl)propionate-2,2,3,3-d4 (0.105 g, 1.2 equivalents) was reacted with an acid chloride prepared from thionyl chloride, 5,6,7,8-tetrahydro-5,5,8,8-tetramethyl-2-naphthylamine (1 equivalent) and triethylamine (1 equivalent) in benzene (4 ml) in the presence of a small amount of DMF (or pyridine) at room temperature for 5 hours. The reaction mixture was washed successively with diluted hydrochloric acid and sodium hydrogencarbonate solution, and dehydrated over anhydrous potassium carbonate, and the solvent was evaporated to obtain the title compound (0.174 g).
H¹-NMR(CDCl₃): δ 1.28 (6H, s), 1.30 (6H, s), 1.69 (4H, s), 2.64 (0.06-0.08, br. C2-H), 2.99 (0.02H, br.s, C1H), 3.68 (3H, s), 7.30 (1H, d, J=8.4Hz), 7.32 (2H, d, J=8.4Hz), 7.42 (1H, dd, J=8.4, 2.4Hz), 7.52 (1H, d, J=2.4Hz), 7.69 (1H, br.s), 7.80 (2H, d, J=8.4Hz) MS (m/z): 397 (M+)

### Example 5: 3-[4-[(5,6,7,8-Tetrahydro-5,5,8,8-tetramethyl-2-naphthaleny)carbamoyl]phenyl]propionic acid-2,2,3,3-d4

The methyl ester (0.17 g) obtained in Example 4 was suspended in ethanol (5 ml), the suspension was added with 2 M NaOH solution (1 ml), and the mixture was stirred at room temperature for 4 hours. The reaction mixture was made acidic by addition of 2 M HCl, and extracted with chloroform. The organic layer was washed with brine, and dried over anhydrous sodium sulfate, then the solvent was evaporated, and the residue was recrystallized from ethyl acetate and hexane to obtain the title compound (122 mg).
H¹NMR (CD₃OD): δ 1.28 (6H, s), 1.30 (6H, s), 1.72 (4H, s), 2.62 (0.06-0.08, br, C2H), 2.96 (0.02H, br, C1H), 7.30 (1H, d, J=8.7Hz), 7.38 (2H, d, J=8.1), 7.43 (1H, dd, J=8.7, 2.1Hz), 7.63 (1H, d, J=2.1Hz), 7.86 (2H, d=8.1)
MS: 383 (M+)

### Example 6: 3-[4-[(5,6,7,8-Tetrahydro-5,5,8,8-tetramethyl-2-naphthalenyl)carbamoyl]phenyl]propionic acid-3,3-d2

A suspension of 4-CD₃-benzoic acid (0.511 g) and NBS (0.784 g) in carbon tetrachloride (10 ml) was added with AIBN (0.121 g), and the mixture was refluxed by heating for 4 hours. The residue was added with 10% aqueous Na₂S₂O₃, and the mixture was extracted with chloroform. The organic layer was washed with brine, and dried over anhydrous sodium sulfate, then the solvent was evaporated, and the residue was recrystallized from methanol and chloroform to obtain a bromide compound (0.530 g).

A solution of acetic acid tert-butyl ester (0.185 ml) in tetrahydrofuran (THF, 1 ml) was cooled at -78°C, and slowly added with LiHMDS (1.0 M in THF, 1.15 ml), and the mixture was stirred for 30 minutes. The reaction mixture was added with a solution of the aforementioned bromide compound (0.100 g) in THF, the reaction was allowed at -78°C for 1 hour, and then the reaction mixture was gradually warmed to room temperature, and stirred overnight. The reaction mixture was added with saturated aqueous ammonium chloride, and the mixture was extracted with ethyl acetate. The organic layer was washed and dried, and then the solvent was evaporated to obtain 3-(4-carboxyphenylpropionic acid-3,3-d2 tert-butyl ester.

The above ester (102 mg) was suspended in benzene (3 ml), the suspension was added with thionyl chloride (0.5 ml), and the mixture was refluxed for 3 hours. The reaction mixture was further added with benzene (5 ml) and pyridine (2 ml), the mixture was added with 5,6,7,8-tetrahydro-5,5,8,8-tetramethyl-2-naphthylamine (0.99 g), and the mixture was left overnight at room temperature. The reaction mixture was made acidic with 2 N HCl, and extracted with ethyl acetate, the organic layer was washed and dried, and then the solvent was evaporated. The residue was subjected to column chromatography (silica gel, 3 g) to obtain a condensate (0.162 g) from a fraction eluted with ethyl acetate and hexane (1:10).

The resulting tert-butyl ester (155 mg) was suspended in dichloromethane (3 ml), and the suspension was added with trifluoroacetic acid (0.5 ml). The mixture was stirred at room temperature for 2 hours, and further added with trifluoroacetic acid (1.5 ml), and the mixture was stirred at room temperature for 3 hours. After the solvent was evaporated, the residue was recrystallized from ethyl acetate and hexane to obtain crystals of the title compound (0.112 g).
H¹NMR (CDCl₃): δ 1.28 (6H, s), 1.30 (6H, s), 1.42 (9H, s), 1.69 (4H, s), 2.71 (2H, s), 7.30 (1H, d, J=8.7Hz), 7.33 (2H, d, J=8.4), 7.42 (1H, dd, J=8.7, 2.1Hz), 7.53 (1H, d, J=2.1Hz), 7.77 (1H, br.s), 7.81 (2H, d, J=8.4Hz)

The deuteration purity was found to be higher than 95% on the basis of the results of NMR.

### Example 7: 3-[4-[(5,6,7,8-Tetrahydro-5,5,8,8-tetramethyl-2-naphthalenyl)carbamoyl]phenyl]propionic acid-2,2-d2

A suspension of 4-[(5,6,7,8-tetrahydro-5,5,8,8-tetramethyl-2-naphthalenyl)-carbamoyl]benzaldehyde (0.125 g) in acetonitrile (3 ml) was added with Meldrum's acid (0.059 g) and Hantzsch ester (0.099 g). The reaction mixture was added with L-proline, the mixture was stirred overnight, and then the solvent was evaporated. The residue was subjected to column chromatography (silica gel, 5 g) to obtain a condensate (0.146 g) from a fraction eluted with ethyl acetate and hexane (1:2). The condensate (0.125 g) was dissolved in dried pyridine (5 ml), the solution was added with deuterated water (0.5 ml), and the mixture was refluxed overnight and then left to cool. The reaction mixture was made acidic with 2 N HCl, and then extracted with chloroform. The organic layer was washed and dehydrated, and then the solvent was evaporated. The residue was recrystallized from ethyl acetate and hexane.
H¹NMR (CDC1₃): δ 1.28 (6H, s), 1.30 (6H, s), 1.69 (4H, s), 3.02 (2H, br.s), 7.30 (1H, d, J=8.4Hz), 7.32(2H, d, J=8.4), 7.42 (1H, dd, J=8.4, 1.8Hz), 7.53 (1H, d, J=1.8), 7.76 (1H, br.s), 7.81 (2H, d, J=8.4)

The deuteration purity was found to be higher than 95% on the basis of the results of NMR.

### Example 8: In vivo kinetic parameters of 3-[4-[(5,6,7,8-tetrahydro-5,5,8,8-tetramethyl-2-naphthalenyl)carbamoyl]phenyl]propionic acid-2,2,3,3-d4 in mouse

6-Week old ddY male mice were starved for 12 hours, and then used for the experiment. To the mice, Am80 (3.30 mg/kg), 3-[4-[(5,6,7,8-tetrahydro-5,5,8,8-tetramethyl-2-naphthalenyl)carbamoyl]phenyl]propionic acid (protium compound, M700, 3.56 mg/kg), or 3-[4-[(5,6,7,8-tetrahydro-5,5,8,8-tetramethyl-2-naphthalenyl)carbamoyl]phenyl]propionic acid-2,2,3,3-d4 (deuterated propionic acid derivative, Y616, 3.60 mg/kg) was orally administered (the dose in terms of molar amount was 9.4 µmol/kg for all the compounds). A 94 mM solution of each compound in DMSO was prepared, and suspended in 0.5% methylcellulose so as to be diluted 100 times, and the suspension was orally administered at a dose of 10 ml/kg by using a 1-ml syringe and a catheter for oral administration.

After 15 minutes, 30 minutes, 1 hour, 2 hours, 3 hours, 6 hours, 12 hours, and 24 hours from the administration of each compound, whole blood was collected from the mouse abdominal aorta under diethyl ether anesthesia by using a heparin-treated glass syringe and a 26G hypodermic needle (five mice were used for each compound). The collected blood was centrifuged at 13,700 x g and 4°C for 5 minutes to separate the plasma, and the separated plasma was stored at -30°C.

The plasma (100 µL) was put into a 1.5-ml tube, and added with ethyl acetate (400 µl), the mixture was stirred, ultrasonicated for 1 minute, and centrifuged at 13,700 x g and 20°C for 3 minutes, and the supernatant was collected. The residue was added with ethyl acetate (400 µl), and the mixture was subjected once again to the aforementioned procedure. The collected supernatant was put into a 1.5-ml tube in an appropriate volume, and dried with nitrogen gas. The residue was dissolved in methanol (100 µl) to obtain a sample for LC/MS.

All the samples were used for LC in a volume of 5 µl. The LC/MS apparatus used was constituted with Waters 2695 Separations Module (Nihon Waters) and 3100 Mass Detector (Nihon Waters). Each compound was quantified by the absolute calibration curve method. The resulting in vivo kinetic parameters are shown in Table 1.

The protium compound and the deuterated propionic acid derivative were metabolically converted into Am80 as the active medicament. The half-life (t1/2) of the prodrug of the deuterated propionic acid derivative was extended, and AUC thereof was also increased. When the deuterated propionic acid derivative was administered, t1/2 of the active medicament Am80 metabolically generated from the prodrug was increased about twice compared with that obtained by administering the protium compound, and increase in AUC was also observed. Further, when the deuterated propionic acid derivative was administered, the metabolic intermediate M1 (4-[(5,6,7,8-tetrahydro-5,5,8,8-tetramethyl-2-naphthalenyl)carbamoyl]phenylcinnamic acid or 2,3-deuterated compound thereof), which was generated in the generation process of the active medicament Am80, markedly decreased, and the metabolic intermediate M2 (3-[4-[(5,6,7,8-tetrahydro-5,5,8,8-tetramethyl-2-naphthalenyl)carbamoyl]phenyl]-3-hydroxypropionic acid or 2,3-deuterated compound thereof) was not observed. M3, which is a 3-keto compound, could not be detected.

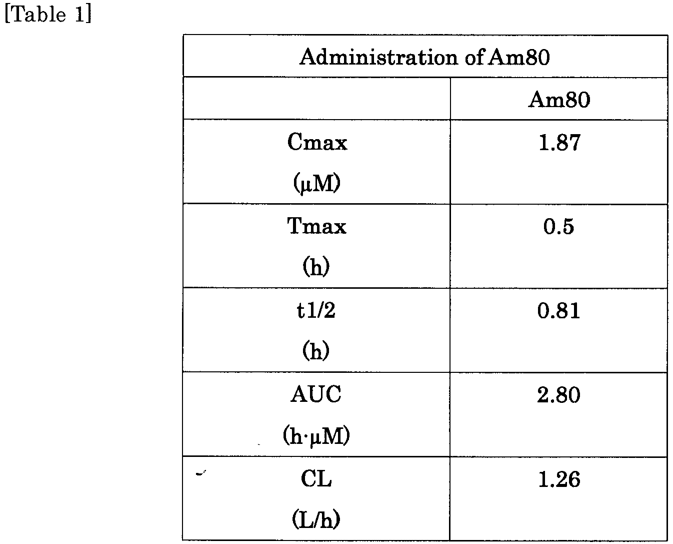

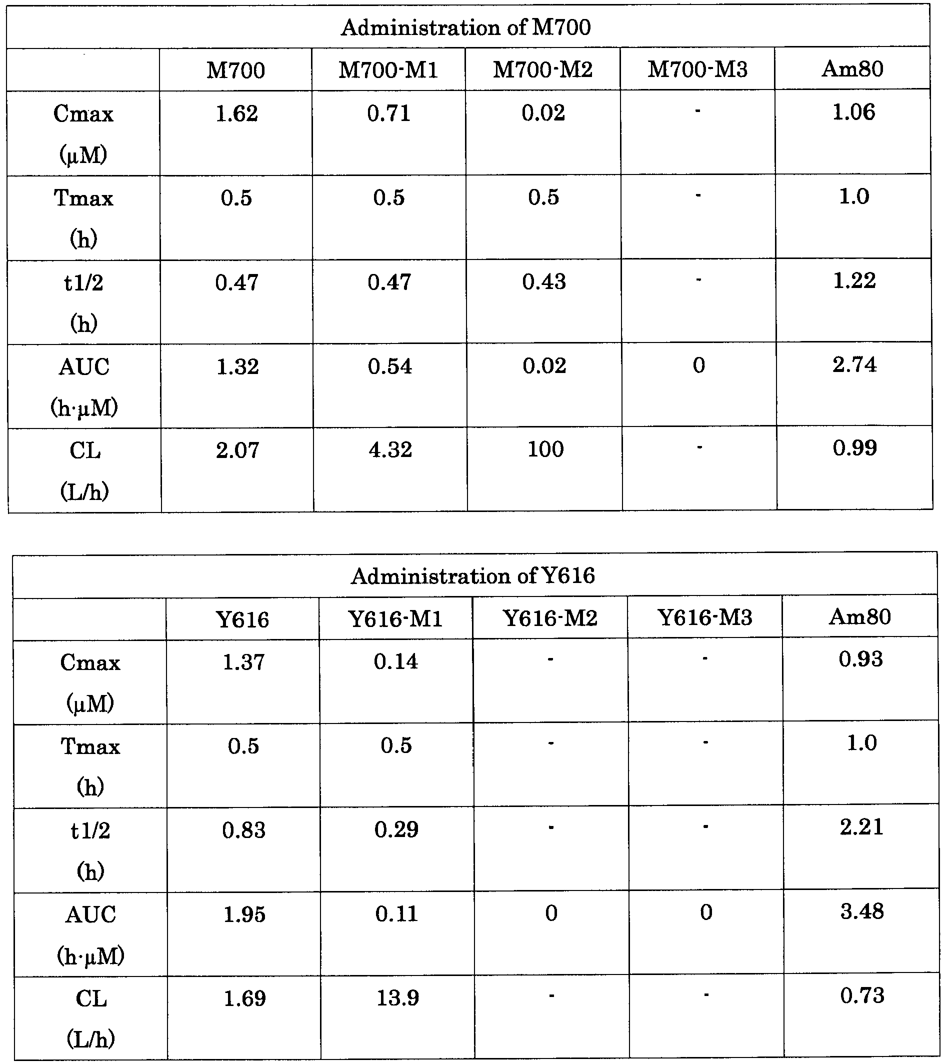

The protium compound and the deuterated propionic acid derivative were metabolically converted into Am80 as the active medicament. The half-life (t1/2) of the prodrug of the deuterated propionic acid derivative was extended, and AUC thereof was also increased. When the deuterated propionic acid derivative was administered, t1/2 of the active medicament Am80 metabolically generated from the prodrug was increased about twice compared with that obtained by administering the protium compound, and increase in AUC was also observed. Further, when the deuterated propionic acid derivative was administered, the metabolic intermediate M1 (4-[(5,6,7,8-tetrahydro-5,5,8,8-tetramethyl-2-naphthalenyl)carbamoyl]phenylcinnamic acid or 2,3-deuterated compound thereof), which was generated in the generation process of the active medicament Am80, markedly decreased, and the metabolic intermediate M2 (3-[4-[(5,6,7,8-tetrahydro-5,5,8,8-tetramethyl-2-naphthalenyl)carbamoyl]phenyl]-3-hydroxypropionic acid or 2,3-deuterated compound thereof) was not observed. M3, which is a 3-keto compound, could not be detected.

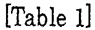

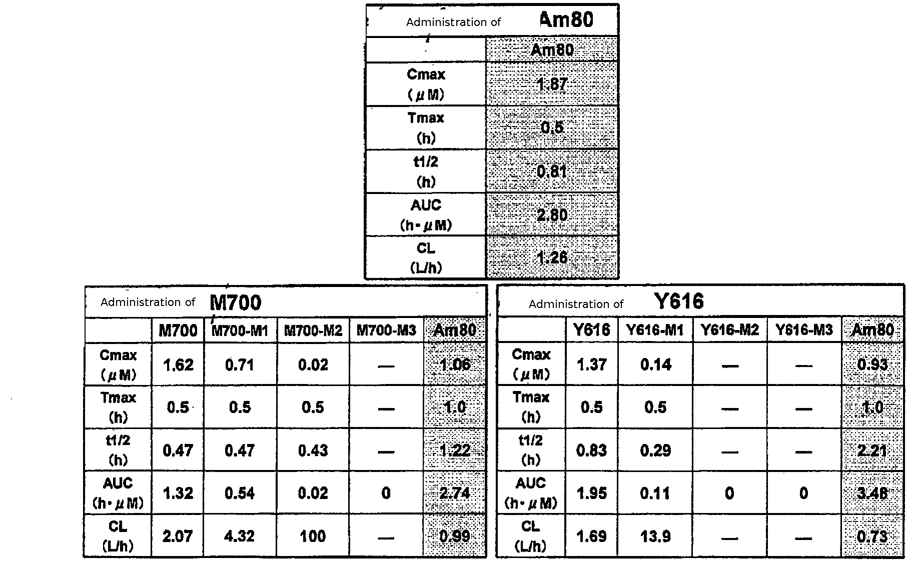

### Industrial Applicability

The deuterated propionic acid derivative provided by the present invention has superior characteristic features as a prodrug concerning the pharmacokinetic parameters such as the maximum blood concentration, blood concentration half-life (t1/2) and area under the blood concentration-time curve (AUC) compared with the protium compound, and therefore it is useful as, for example, a prodrug that can continuously exhibit the actions of Am80 as an active medicament over a long period of time.

## Claims

1. A compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof.

2. 3-[4-[(5,6,7,8-Tetrahydro-5,5,8,8-tetramethyl-2-naphthalenyl)carbamoyl]-phenyl]propionic acid-2,2,3,3-d4, 3-[4-[(5,6,7,8-tetrahydro-5,5,8,8-tetramethyl-2-naphthalenyl)carbamoyl]phenyl]propionic acid-2,2-d2, 3-[4-[(5,6,7,8-tetrahydro-5,5,8,8-tetramethyl-2-naphthalenyl)carbamoyl]phenyl]propionic acid-3,3-d2, a salt thereof, or an ester thereof.

3. A prodrug for releasing Am80 after being absorbed into a living body, which comprises a compound corresponding to 3-[4-[(5,6,7,8-tetrahydro-5,5,8,8-tetramethyl-2-naphthalenyl)carbamoyl]phenyl]propionic acid in which a part or all of hydrogen atoms in the ethylene group constituting the propionic acid moiety are replaced with deuterium atoms, a salt thereof, or an ester thereof as an active ingredient.
